# EUROPEAN PATENT APPLICATION

(11) **EP 4 806 364 A1**
(43) Date of publication of application: **16.09.2026**
(21) Application number: 26163084.2
(22) Date of filing: 09.03.2026
(51) Int. Cl.: A61B 6/40, A61B 6/00, H05G 1/32

(54) **TUBE VOLTAGE ADJUSTMENT METHOD AND APPARATUS, X-RAY SYSTEM, AND COMPUTER-READABLE STORAGE MEDIUM**

(30) Priority: 10.03.2025 CN 202510277991
(71) Applicant: Siemens Healthineers AG, 91301 Forchheim (DE)
(72) Inventor: XU, Yun Tao, Shanghai, SH, 201318 (CN)
(74) Representative: HKW Intellectual Property PartG mbB

(57) **Abstract**

Embodiments of the present invention disclose a tube voltage adjustment method and apparatus, an X-ray system, and a computer-readable storage medium. The method comprises: at the end of a present exposure, obtaining an X-ray image acquired by the present exposure, and obtaining an average grayscale of the X-ray image; obtaining a first ratio between the average grayscale and a target grayscale of this X-ray examination; obtaining an adjustment direction and an adjustment extent of tube voltage of an X-ray tube according to the first ratio; and adjusting a present tube voltage according to the adjustment direction and adjustment extent of tube voltage of the X-ray tube. Embodiments of the present invention increase the speed of X-ray examination.

## Description

### TECHNICAL FIELD

The present invention relates to the technical field of medical imaging, in particular a method and apparatus for adjustment of tube voltage in X-ray examinations, an X-ray system, and a computer-readable storage medium.

### BACKGROUND ART

There are two main types of exposure in mobile C-arm X-ray systems: DR (Digital Radiography, digital X-ray) exposure and fluoroscopy exposure. DR exposure is generally used to capture static images; fluoroscopy exposure is used for dynamic continuous imaging.

To obtain high-quality X-ray images, the emitted X-rays must attain the target dose on each occasion that an X-ray examination is performed. The target dose is defined by a parameter set of an acquisition mode (e.g. DR, fluoroscopy) combined with a user-selected general-purpose dose level (low, medium or high).

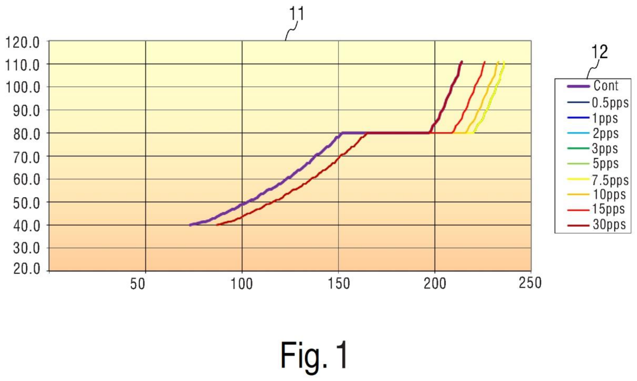

At present, organ program curves for X-ray examination are respectively defined for each type of organ. For example: an organ program curve for a particular type of organ defines all tube voltages that can be used for an X-ray examination of that type of organ, as well as tube currents and exposure times for each tube voltage. Fig. 1 is an exemplary drawing of organ program curves for different frame rates defined for a particular type of organ. In the figure, 11 denotes the organ program curves for different frame rates defined for a particular type of organ, the vertical coordinate is the tube voltage that can be used, and the horizontal coordinate is the sequence number of the tube voltage that can be used. 12 denotes the frame rates corresponding to the curves of different colors in 11; "cont" means continuous frames, and PPS (Pulse Per Second) is the unit of frame rate, meaning the number of pulses per second. It should be explained that 11 actually includes curves for the 10 frame rates shown in 12, but only a few curves can be clearly seen in 11 due to coincidence of curves. In addition, the tube voltages defined in the organ program curves are discrete rather than continuous, and the organ program curves are produced from the discrete tube voltages by fitting. In actual applications, each curve corresponds to a table, which defines each tube voltage included in the curve and the corresponding tube current and exposure time.

When an X-ray examination begins, exposure parameters (tube voltage, tube current and exposure time, etc.) for the first exposure are calculated according to the target dose for this examination and a water value obtained in a prior acquisition sequence, wherein, if there is no prior acquisition sequence, a 20 cm water equivalent value is used. These exposure parameters are then used to perform exposure, and an actual dose is calculated according to an acquired X-ray image. If the actual does has not reached the target dose, a tube voltage is reselected according to the organ program curve to perform exposure, until the actual dose reaches the target dose. The reselection of tube voltage is done according to a fixed number of jumps, i.e. the third nearest tube voltage to the present actual tube voltage in the organ program curve is generally selected as the new tube voltage. This method has the following drawback: since each adjustment of tube voltage is based on a fixed number of jumps, multiple adjustments are necessary to achieve proper adjustment if the actual dose is far away from the target dose. This will prolong the time needed to obtain a correct X-ray image, especially in the case of DR exposure.

### SUMMARY OF THE INVENTION

In view of the above, one aspect of embodiments of the present invention proposes a tube voltage adjustment method and apparatus, to increase the X-ray examination speed; another aspect of embodiments of the present invention proposes an X-ray system, to increase the X-ray examination speed; and a further aspect of embodiments of the present invention proposes a computer-readable storage medium, to increase the X-ray examination speed.

A tube voltage adjustment method, comprising:
at the end of a present exposure, obtaining an X-ray image acquired by the present exposure, and obtaining an average grayscale of the X-ray image;
obtaining a first ratio between the average grayscale and a target grayscale of this X-ray examination;
obtaining an adjustment direction and an adjustment extent of tube voltage of an X-ray tube according to the first ratio;
adjusting a present tube voltage according to the adjustment direction and adjustment extent of tube voltage of the X-ray tube.

The step of obtaining an average grayscale of the X-ray image comprises:
removing pixel points lower than a first grayscale value and higher than a second grayscale value in the X-ray image, calculating an average grayscale of remaining pixel points, and taking the average grayscale thus obtained to be the average grayscale of the X-ray image;
wherein the first grayscale is the grayscale of a pixel point whose sequenced position is equal to an a% position when all pixel points of the X-ray image are sequenced from low to high grayscale, and the second grayscale is the grayscale of a pixel point whose sequenced position is equal to a b% position when all pixel points of the X-ray image are sequenced from low to high grayscale; a and b are preset values, and a < b.

The step of obtaining an adjustment direction and an adjustment extent of tube voltage of an X-ray tube according to the first ratio comprises:
when the first ratio is greater than 1, determining the adjustment direction of tube voltage of the X-ray tube to be: decreasing the present tube voltage;
when the first ratio is less than 1, determining the adjustment direction of tube voltage of the X-ray tube to be: increasing the present tube voltage;
and the greater the distance between the first ratio and 1, the greater the adjustment extent of the present tube voltage.

The step of obtaining an adjustment direction and an adjustment extent of tube voltage of an X-ray tube according to the first ratio comprises:
performing a logarithm operation on the first ratio, and obtaining an adjustment step length of the present tube voltage according to the principle that an adjustment step length of tube voltage and a result of the logarithm operation on the first ratio form an inverse linear relationship;
or comprises:
   calculating a second ratio between a set tube current of the present exposure and an actual tube current of the present exposure, performing a logarithm operation on the product of the first ratio and the second ratio, and obtaining an adjustment step length of the present tube voltage according to the principle that an adjustment step length of tube voltage and a result of the logarithm operation on the product form an inverse linear relationship.

The step of obtaining an adjustment direction and an adjustment extent of tube voltage of an X-ray tube according to the first ratio comprises:
obtaining a present base adjustment extent according to the present tube voltage, obtaining a present correction factor according to the first ratio, and obtaining an adjustment step length of the present tube voltage according to the present base adjustment extent and the present correction factor.

The step of obtaining a present base adjustment extent according to the present tube voltage comprises:
if the present tube voltage is less than a first voltage threshold, calculating the difference between the present tube voltage and a tube voltage lower limit less 1, and obtaining the present base adjustment extent according to the principle that the present base adjustment extent and a result of a logarithm operation on the difference form a positive linear relationship;
if the present tube voltage is greater than or equal to the first voltage threshold and less than a second voltage threshold, obtaining the present base adjustment extent according to the principle that the present base adjustment extent and the present tube voltage form a positive linear relationship;
if the present tube voltage is greater than or equal to the second voltage threshold, taking the difference between a tube voltage upper limit and the second voltage threshold to be the present base adjustment extent.

The step of obtaining a present correction factor according to the first ratio comprises:
performing a logarithm operation on the first ratio, and obtaining the present correction factor according to the principle that the present correction factor and a result of the logarithm operation on the first ratio form an inverse linear relationship;
or comprises
calculating a second ratio between a set tube current of the present exposure and an actual tube current of the present exposure, performing a logarithm operation on the product of the first ratio and the second ratio, and obtaining the present correction factor according to the principle that the present correction factor and a result of the logarithm operation on the product form an inverse linear relationship.

The step of obtaining an adjustment step length of the present tube voltage according to the present base adjustment extent and the present correction factor comprises:
taking the product of the present base adjustment extent and the present correction factor to be the adjustment step length of the present tube voltage.

After the end of the present exposure, before the step of obtaining the average grayscale of the X-ray image, the method further comprises:
judging whether the first ratios corresponding to a preset number of most recent consecutive exposures are all within [1 - *c%,* 1 + *c*%, and if so, determining that a tube voltage adjustment process for this X ray examination ends, wherein *c* is a preset value.

A tube voltage adjustment apparatus, comprising:
an image grayscale acquisition module, configured to: at the end of a present exposure, obtain an X-ray image acquired by the present exposure, obtain an average grayscale of the X-ray image, and obtain a first ratio between the average grayscale and a target grayscale of this X-ray examination;
a tube voltage adjustment module, configured to: obtain an adjustment direction and an adjustment extent of tube voltage of an X-ray tube according to the first ratio; and adjust a present tube voltage according to the adjustment direction and adjustment extent of tube voltage of the X-ray tube.

The tube voltage adjustment module obtaining an adjustment direction and an adjustment extent of tube voltage of an X-ray tube according to the first ratio comprises:
when the first ratio is greater than 1, determining the adjustment direction of tube voltage of the X-ray tube to be: decreasing the present tube voltage;
when the first ratio is less than 1, determining the adjustment direction of tube voltage of the X-ray tube to be: increasing the present tube voltage;
and the greater the distance between the first ratio and 1, the greater the adjustment extent of the present tube voltage.

The tube voltage adjustment module obtaining an adjustment direction and an adjustment extent of tube voltage of an X-ray tube according to the first ratio comprises:
performing a logarithm operation on the first ratio, and obtaining an adjustment step length of the present tube voltage according to the principle that an adjustment step length of tube voltage and a result of the logarithm operation on the first ratio form an inverse linear relationship;
or comprises:
   calculating a second ratio between a set tube current of the present exposure and an actual tube current of the present exposure, performing a logarithm operation on the product of the first ratio and the second ratio, and obtaining an adjustment step length of the present tube voltage according to the principle that an adjustment step length of tube voltage and a result of the logarithm operation on the product form an inverse linear relationship.

The tube voltage adjustment module obtaining an adjustment direction and an adjustment extent of tube voltage of an X-ray tube according to the first ratio comprises:
obtaining a present base adjustment extent according to the present tube voltage, obtaining a present correction factor according to the first ratio, and obtaining an adjustment step length of the present tube voltage according to the present base adjustment extent and the present correction factor.

The tube voltage adjustment module obtaining a present base adjustment extent according to the present tube voltage comprises:
if the present tube voltage is less than a first voltage threshold, calculating the difference between the present tube voltage and a tube voltage lower limit less 1, and obtaining the present base adjustment extent according to the principle that the present base adjustment extent and a result of a logarithm operation on the difference form a positive linear relationship;
if the present tube voltage is greater than or equal to the first voltage threshold and less than a second voltage threshold, obtaining the present base adjustment extent according to the principle that the present base adjustment extent and the present tube voltage form a positive linear relationship;
if the present tube voltage is greater than or equal to the second voltage threshold, taking the difference between a tube voltage upper limit and the second voltage threshold to be the present base adjustment extent.

The tube voltage adjustment module obtaining a present correction factor according to the first ratio comprises:
performing a logarithm operation on the first ratio, and obtaining the present correction factor according to the principle that the present correction factor and a result of the logarithm operation on the first ratio form an inverse linear relationship;
or comprises:
   calculating a second ratio between a set tube current of the present exposure and an actual tube current of the present exposure, performing a logarithm operation on the product of the first ratio and the second ratio, and obtaining the present correction factor according to the principle that the present correction factor and a result of the logarithm operation on the product form an inverse linear relationship.

After the end of the present exposure, before obtaining the average grayscale of the X-ray image, the image grayscale acquisition module is further configured to:
judge whether the first ratios corresponding to a preset number of most recent consecutive exposures are all within [1 - *c%,* 1 + *c*%], and if so, determining that a tube voltage adjustment process for this X ray examination ends, wherein *c* is a preset value.

An X-ray system, comprising the tube voltage adjustment apparatus as described in any one of the embodiments above.

A computer-readable storage medium, storing an instruction which, when executed by a processor, executes the steps of the tube voltage adjustment method as described in any one of the embodiments above.

In embodiments of the present invention, at the end of each exposure, the ratio between the average grayscale of the acquired X-ray image and the target grayscale is obtained, an adjustment direction and adjustment extent of tube voltage of the X-ray tube are obtained according to the ratio, and the present tube voltage is adjusted according to the adjustment direction and adjustment extent of tube voltage of the X-ray tube. Thus, the adjustment direction and adjustment extent of tube voltage can be adapted to the grayscale of the X-ray image, increasing the speed of X-ray examination.

### BRIEF DESCRIPTION OF THE DRAWING

Preferred embodiments of the present invention will be described in detail below with reference to the drawings, to give an ordinary person skilled in the art a clearer understanding of the abovementioned and other features and advantages of the present invention. In the drawings:
Fig. 1 is an exemplary drawing of organ program curves for different frame rates defined for a particular type of organ.
Fig. 2 is a flow chart of a tube voltage adjustment method provided in an embodiment of the present invention.
Fig. 3 is a schematic drawing of a grayscale histogram of an X-ray image acquired in a particular exposure of a particular X-ray examination in an application example of the present invention.
Fig. 4 is a schematic drawing of the relationship between *U_{curr}* and *U_{basₐₚₘ}* in an X-ray examination of an organ.
Fig. 5 is a schematic drawing of the relationship between *x* and *Corr_{fac}* in an X-ray examination of an organ.
Fig. 6 is a graph of the change in average grayscale of an image when a 23 cm phantom is subjected to exposure using the prior art and the present invention, respectively.
Fig. 7 is a graph of the change in average grayscale of an image when a 31 cm orthopedic anteroposterior phantom is subjected to exposure using the prior art and the present invention, respectively.
Fig. 8 is a structural schematic drawing of a tube voltage adjustment apparatus provided in an embodiment of the present invention.

### KEY TO THE DRAWINGS

| Label | Meaning |
|---|---|
| 11 | Organ program curves for different frame rates defined for a particular type of organ |
| 12 | Frame rates corresponding to curves of different colors in 11 |
| 201-204 | Steps |
| 31 | First grayscale |
| 32 | Second grayscale |
| 61 | Curve of change in average grayscale of image corresponding to prior art |
| 62 | Curve of change in average grayscale of image corresponding to present invention |
| 71 | Curve of change in average grayscale of image corresponding to prior art |
| 72 | Curve of change in average grayscale of image corresponding to present invention |
| 80 | Tube voltage adjustment apparatus |
| 81 | Image grayscale acquisition module |
| 82 | Tube voltage adjustment module |

### DETAILED DESCRIPTION OF EMBODIMENTS

To clarify the objective, technical solution and advantages of the present invention, the present invention is explained in further detail below by way of embodiments.

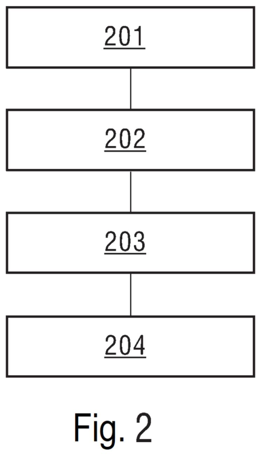

Fig. 2 is a flow chart of a tube voltage adjustment method provided in an embodiment of the present invention. As shown in Fig. 2, the method comprises the following specific steps:
Step 201: at the end of a present exposure, obtaining an X-ray image acquired by the present exposure, and obtaining an average grayscale of the X-ray image.

When an X-ray examination begins, firstly, based on an organ program curve for an organ examined on this occasion, a tube voltage is selected from tube voltages defined in the curve, to serve as a tube voltage of a first exposure. In this embodiment, generally, a middle value of tube voltages defined in the organ program curve is selected as the tube voltage of the first exposure. For example: the tube voltages defined in the organ program curve for the organ examined on this occasion have a minimum value of 40 kV (kilovolts) and a maximum value of 110 kV, so 75 kV is selected as the tube voltage of the first exposure. At the same time, it can be ascertained from the organ program curve that the tube current and exposure time corresponding to 75 kV are respectively 24 mA (milliamps) and 39.8 ms (milliseconds). Thus, the first exposure is performed using 75 kV, 24 mA and 39.8 ms.

In an optional embodiment, in this step 201, obtaining an average grayscale of the X-ray image comprises: removing pixel points lower than a first grayscale value and higher than a second grayscale value in the X-ray image, calculating an average grayscale of the remaining pixel points, and taking the average grayscale thus obtained to be the average grayscale of the X-ray image, wherein the first grayscale is the grayscale of a pixel point whose sequenced position is equal to an a% position when all pixel points of the X-ray image are sequenced from low to high grayscale, and the second grayscale is the grayscale of a pixel point whose sequenced position is equal to a b% position when all pixel points of the X-ray image are sequenced from low to high grayscale. a and b are preset values, and a < b. Typically, 0 < a% ≤ 5%, 75% ≤ b% ≤ 85%; preferably, a% = 3%, b% = 80%. Pixel points lower than the first grayscale value are generally considered to be metal points on metal material (such as nails, pacemakers, etc.) implanted into the human body (metal absorbs radiation, so has a low grayscale). Pixel points higher than the second grayscale value are generally considered to be overexposure points. Both are interfering points, so should be filtered out. If calculation of the sequence numbers corresponding to the a% and b% positions results in a decimal, the calculation result is rounded down.

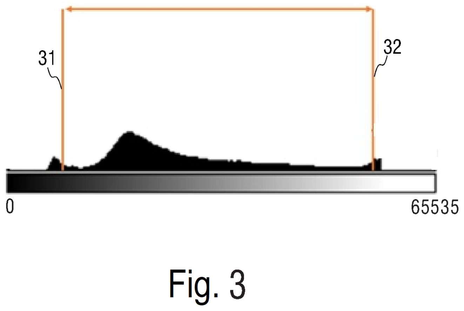

Fig. 3 is a schematic drawing of a grayscale histogram of an X-ray image acquired in a particular exposure of a particular X-ray examination in an application example of the present invention. In this example, let a% = 3%, b% = 80%, and the image size be 1024*1024. The 1024*1024 pixel points of the X-ray image are sequenced from low to high grayscale value; when sequencing is complete, the grayscale of the floor(1024*1024*3%)th pixel point is taken to be the first grayscale, and the grayscale of the floor(1024*1024*80%)th pixel point is taken to be the second grayscale, then the mean value of the grayscale values of all pixel points with grayscale values within [first grayscale, second grayscale] in the X-ray image is found. This mean value is the average grayscale of the X-ray image; floor() is a rounding-down function. In Fig. 3, 31 is the first grayscale, and 32 is the second grayscale.

Step 202: obtaining a first ratio between the average grayscale in step 201 and a target grayscale of this X-ray examination.

The target grayscale of this X-ray examination = target dose of this X-ray examination * sensitivity of detector. The target dose of this X-ray examination is defined in the organ program curve for the organ examined on this occasion.

Step 203: obtaining an adjustment direction and an adjustment extent of tube voltage of an X-ray tube according to the first ratio.

Step 204: adjusting a present tube voltage according to the adjustment direction and adjustment extent of tube voltage of the X-ray tube.

The next exposure can then be performed according to the adjusted present tube voltage.

In the embodiment above, at the end of each exposure, the ratio between the average grayscale of the acquired X-ray image and the target grayscale is obtained, an adjustment direction and adjustment extent of tube voltage of the X-ray tube are obtained according to the ratio, and the present tube voltage is adjusted according to the adjustment direction and adjustment extent of tube voltage of the X-ray tube. Thus, the adjustment direction and adjustment extent of tube voltage can be adapted to the grayscale of the X-ray image, increasing the speed of X-ray examination.

In an optional embodiment, in step 203, obtaining an adjustment direction and an adjustment extent of tube voltage of an X-ray tube according to the first ratio comprises: when the first ratio is greater than 1, determining the adjustment direction of tube voltage of the X-ray tube to be: decreasing the present tube voltage; when the first ratio is less than 1, determining the adjustment direction of tube voltage of the X-ray tube to be: increasing the present tube voltage; and the greater the distance between the first ratio and 1, the greater the adjustment extent of the present tube voltage.

In the embodiment above, when the first ratio is greater than 1, i.e. when the average grayscale of the X-ray image is higher than the target grayscale, the present tube voltage is decreased; when the first ratio is less than 1, i.e. when the average grayscale of the X-ray image is lower than the target grayscale, the present tube voltage is increased; and the greater the distance between the first ratio and 1, i.e. the greater the difference between the average grayscale of the X-ray image and the target grayscale, the greater the adjustment extent of the present tube voltage. Therefore: the adjustment direction and adjustment extent of tube voltage can be completely adapted to the difference between the average grayscale of the X-ray image and the target grayscale, increasing the speed with which an X-ray image with accurate brightness can be obtained, i.e. increasing the speed of X-ray examination.

In an optional embodiment, in step 203, obtaining an adjustment direction and an adjustment extent of tube voltage of an X-ray tube according to the first ratio comprises: performing a logarithm operation on the first ratio, and obtaining an adjustment step length of the present tube voltage according to the principle that an adjustment step length of tube voltage and a result of the logarithm operation on the first ratio form an inverse linear relationship.

For example: *y = e * lnx + f.* Here, *ln* is the natural logarithm operation symbol, *xis* the first ratio, *y* is the adjustment step length of tube voltage, *e* and *f* are preset constants, and *e <* 0. The specific values of e and *f* can be determined on the basis of multiple tests; typically, -5 ≤ *e* < 0, 0 ≤ *f* ≤ 10.

Once *y* has been calculated, the tube voltage *Uₙₑₓₜ* of the next exposure is calculated according to *Uₙₑₓₜ* = *U_{curr} + y,* where *U_{curr}* is the tube voltage of the present exposure. It should be explained that *U_{curr}* and *Uₙₑₓₜ* both refer to tube voltages defined in the organ program curve for the organ examined on this occasion; if the value of the sum obtained from *U_{curr} + y* does not have a corresponding tube voltage in the organ program curve, the tube voltage closest to *U_{curr} + y* is sought in the organ program curve, to serve as the tube voltage of the next exposure.

In the embodiment above, firstly, the logarithm operation is performed on the first ratio, then the adjustment step length of the present tube voltage is obtained according to the principle that the adjustment step length of tube voltage and the result of the logarithm operation on the first ratio form an inverse linear relationship. Thus: when the first ratio is greater than 1, i.e. when the average grayscale of the X-ray image is higher than the target grayscale, the present tube voltage is decreased; when the first ratio is less than 1, i.e. when the average grayscale of the X-ray image is lower than the target grayscale, the present tube voltage is increased; and if the distance between the first ratio and 1 is greater, i.e. if the difference between the average grayscale of the X-ray image and the target grayscale is greater, the adjustment extent of the present tube voltage increases logarithmically. Therefore: the adjustment direction and adjustment extent of tube voltage can be completely adapted to the difference between the average grayscale of the X-ray image and the target grayscale, increasing the speed with which an X-ray image with accurate brightness can be obtained, i.e. increasing the speed of X-ray examination.

The following is taken into account: in actual applications, there might be a difference between an actual tube current of the X-ray tube and a set tube current, and if the difference is large, the grayscale of the X-ray image will be affected. Thus, to increase the speed with which an X-ray image with accurate brightness can be obtained, the following optimized solution is proposed:
In an optional embodiment, in step 203, obtaining an adjustment direction and an adjustment extent of tube voltage of an X-ray tube according to the first ratio comprises: calculating a second ratio between a set tube current of the present exposure and an actual tube current of the present exposure, performing a logarithm operation on the product of the first ratio and the second ratio, and obtaining an adjustment step length of the present tube voltage according to the principle that an adjustment step length of tube voltage and a result of the logarithm operation on the product form an inverse linear relationship.

For example: $y = e ∗ ln \left(\frac{{I}_{{curr}_{set}}}{{I}_{{curr}_{act}}}∗x\right) + f$*.* Here, ln is the natural logarithm operation symbol; *x* is the first ratio; *y* is the adjustment step length of tube voltage; *I_{currₛₑₜ}* is the set tube current of the present exposure, i.e. the tube current corresponding to the tube voltage of the present exposure defined in the organ program curve for the organ examined on this occasion; *I_{curr_{act}}* is the actual tube current of the present exposure; *e* and *f* are preset constants, and *e <* 0. The specific values of *e* and *f* can be determined on the basis of multiple tests; typically, -5 ≤ *e* < 0, 0 ≤ *f* ≤ 10.

Once *y* has been calculated, the tube voltage *Uₙₑₓₜ* of the next exposure is calculated according to *Uₙₑₓₜ* = *U_{curr} + y,* where *U_{curr}* is the tube voltage of the present exposure. It should be explained that *U_{curr}* and *Uₙₑₓₜ* both refer to tube voltages defined in the organ program curve for the organ examined on this occasion; if the value of the sum obtained from *U_{curr} + y* does not have a corresponding tube voltage in the organ program curve, the tube voltage closest to *U_{curr} + y* is sought in the organ program curve, to serve as the tube voltage of the next exposure.

Having conducted multiple tests, and observed and analysed the testing procedures and results, the inventors have found that: the tube voltage of each exposure also affects the adjustment extent of tube voltage. Thus, the following optimized solution is proposed:
In an optional embodiment, in step 203, obtaining an adjustment direction and an adjustment extent of tube voltage of an X-ray tube according to the first ratio comprises: obtaining a present base adjustment extent according to the present tube voltage, obtaining a present correction factor according to the first ratio, and obtaining an adjustment step length of the present tube voltage according to the present base adjustment extent and the present correction factor.

The inventors have found through observation and analysis that: when the tube voltage lies between a tube voltage lower limit and a first voltage threshold, the tube voltage adjustment effect is optimal if a logarithmic relationship exists between the adjustment extent of tube voltage and the distance between the tube voltage and the tube voltage lower limit; when the tube voltage lies between the first voltage threshold and a second voltage threshold, the tube voltage adjustment effect is optimal if a positive linear relationship exists between the adjustment extent of tube voltage and the tube voltage. Thus, the following optimized solution is proposed:
In an optional embodiment, in step 203, obtaining a present base adjustment extent according to the present tube voltage comprises: if the present tube voltage is less than a first voltage threshold, calculating the difference between the present tube voltage and a tube voltage lower limit less 1, and obtaining the present base adjustment extent according to the principle that the present base adjustment extent and a result of a logarithm operation on the difference form a positive linear relationship; if the present tube voltage is greater than or equal to the first voltage threshold and less than a second voltage threshold, obtaining the present base adjustment extent according to the principle that the present base adjustment extent and the present tube voltage form a positive linear relationship; if the present tube voltage is greater than or equal to the second voltage threshold, taking the difference between a tube voltage upper limit and the second voltage threshold to be the present base adjustment extent.

For example: ${U}_{{bas}_{apm}} = \left\{\begin{matrix}m ∗ {e}^{n ∗ \left({U}_{curr}-\left({U}_{min}-1\right)\right) ,} & {U}_{curr} < {U}_{th 1} \\ p ∗ {U}_{curr} + q , & {U}_{th 1} \leq {U}_{curr} < {U}_{th 2} \\ {U}_{max} - {U}_{th 2} , & {U}_{curr} \geq {U}_{th 2}\end{matrix}\right.$

Here, *U_{basₐₚₘ}* is the present base adjustment extent; *U_{curr}* is the tube voltage of the present exposure; *Uₘᵢₙ* is the tube voltage lower limit, i.e. the minimum tube voltage defined in an organ examination curve for the organ examined on this occasion; *Uₘₐₓ* is the tube voltage upper limit, i.e. the maximum tube voltage defined in the organ examination curve for the organ examined on this occasion; *Uₜₕ₁* and *Uₜₕ₂* are respectively the first voltage threshold and the second voltage threshold, where the specific values of *Uₜₕ₁* and *Uₜₕ₂* can be determined on the basis of multiple tests; *m, n, p* and *q* are constants, and *m* > 0, *n* > 0, *p* > 0. The specific values of *m, n, p* and *q* can be determined on the basis of multiple tests; typically, 0 < *m* ≤20, 0 *< n* ≤20, 0 < *p* ≤20, 0 < *q* ≤20. In an optional embodiment, *Uₘᵢₙ =* 40kV, *Uₘₐₓ =* 110 kV, *Uₜₕ₁* = 90 kV, *Uₜₕ₂* = 100 kV.

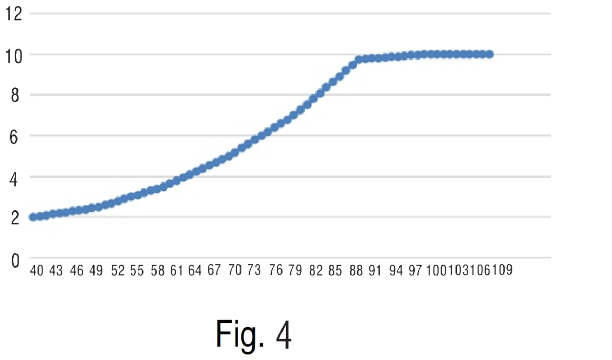

Fig. 4 is a schematic drawing of the relationship between *U_{curr}* and *U_{basₐₚₘ}* in an X-ray examination of an organ. Here, the horizontal coordinate is *Ucurr,* the vertical coordinate is *U_{basₐₚₘ},* and the horizontal and vertical coordinates are both in units of kV, wherein *Uₘᵢₙ =* 40kV, *Uₘₐₓ =* 110kV, *Uₜₕ₁* = 90kV and *Uₜₕ₂* = 100kV.

In an optional embodiment, obtaining a present correction factor according to the first ratio comprises: performing a logarithm operation on the first ratio, and obtaining the present correction factor according to the principle that the present correction factor and a result of the logarithm operation on the first ratio form an inverse linear relationship.

For example: *Corr_{fac}* = *u * lnx + v.* Here, *Corrfac* is the present correction factor; ; *ln* is the natural logarithm operation symbol; *x* is the first ratio; *u* and *v* are constants, and *u* < 0. The specific values of *u* and *v* can be determined on the basis of multiple tests; typically, -5 ≤ *u* < 0, 0 ≤ *v* ≤ 10.

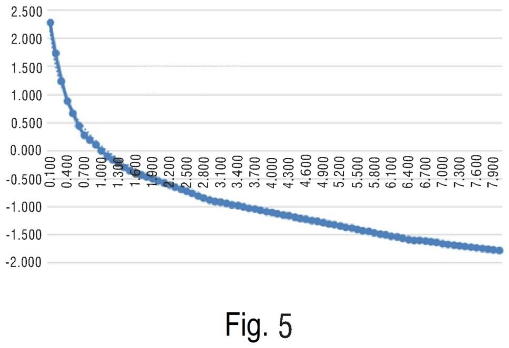

Fig. 5 is a schematic drawing of the relationship between *x* and *Corr_{fac}* in an X-ray examination of an organ. Here, the horizontal coordinate is *x*, and the vertical coordinate is *Corr_{fac}.*

In an optional embodiment, obtaining a present correction factor according to the first ratio comprises: calculating a second ratio between a set tube current of the present exposure and an actual tube current of the present exposure, performing a logarithm operation on the product of the first ratio and the second ratio, and obtaining the present correction factor according to the principle that the present correction factor and a result of the logarithm operation on the product form an inverse linear relationship.

For example: ${Corr}_{fac} = u ∗ ln \left(\frac{{I}_{{curr}_{set}}}{{I}_{{curr}_{act}}}∗x\right) + v$. Here, *Corrfac* is the present correction factor; *ln* is the natural logarithm operation symbol; *x* is the first ratio; *I_{currₛₑₜ}* is the set tube current of the present exposure, i.e. the tube current corresponding to the tube voltage of the present exposure defined in the organ program curve for the organ examined on this occasion; *I_{curr_{act}}* is the actual tube current of the present exposure; *u* and *v* are constants, and *u <* 0. The specific values of *u* and *v* can be determined on the basis of multiple tests; typically, -5 ≤ *u* < 0, 0 ≤ *v* ≤ 10.

In an embodiment, obtaining an adjustment step length of the present tube voltage according to the present base adjustment extent and the present correction factor comprises: taking the product of the present base adjustment extent and the present correction factor to be the adjustment step length of the present tube voltage.

In embodiments of the present invention, for an X-ray examination, when the first ratios corresponding to a preset number of most recent consecutive exposures are all within a preset range in the vicinity of 1, it is determined that no further adjustment of tube voltage is necessary; an X-ray image with accurate brightness can already be obtained using the present tube voltage. That is, in step 201, after the end of the present exposure, before obtaining the average grayscale of the X-ray image, the method further comprises: judging whether the first ratios corresponding to a preset number of most recent consecutive exposures are all within [1 - *c%,* 1 + *c*%], and if so, determining that a tube voltage adjustment process for this X ray examination ends, wherein *c* is a preset value. The specific value of *c* can be determined on the basis of multiple tests; typically, 6% ≤ *c*% ≤ 12%, and preferably, *c*% = 10%.

It should be explained that the tube voltage adjustment method provided in embodiments of the present invention is suitable for C-arm X-ray systems, e.g. mobile C-arm X-ray systems.

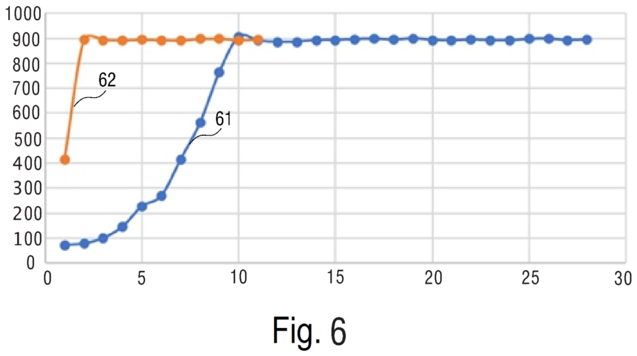

Fig. 6 is a graph of the change in average grayscale of an image when a 23 cm phantom is subjected to exposure using the prior art and the present invention, respectively. The horizontal coordinate is the number of exposures; the vertical coordinate is the average grayscale of the image. The blue curve 61 corresponds to the prior art; the orange curve 62 corresponds to the present invention. Each point on 61 and 62 corresponds to one exposure. As can be seen, 61 only reaches the target grayscale at the 11th exposure, whereas 62 reaches the target grayscale at the 3rd exposure. It is clear that the use of the present invention considerably increases the speed with which an X-ray image with accurate brightness can be obtained.

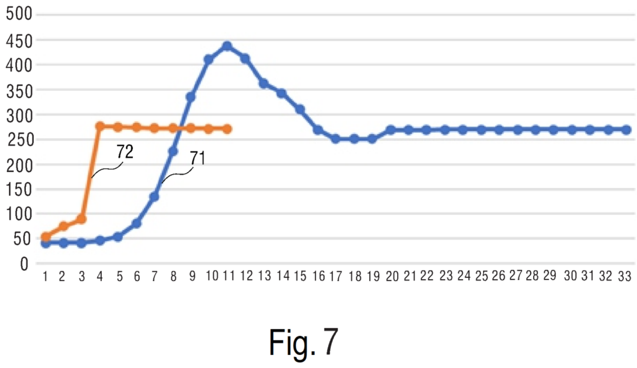

Fig. 7 is a graph of the change in average grayscale of an image when a 31 cm orthopedic anteroposterior phantom is subjected to exposure using the prior art and the present invention, respectively. The horizontal coordinate is the number of exposures; the vertical coordinate is the average grayscale of the image. The blue curve 71 corresponds to the prior art; the orange curve 72 corresponds to the present invention. Each point on 71 and 72 corresponds to one exposure. As can be seen, 71 only reaches the target grayscale at the 17th exposure, whereas 72 substantially reaches the target grayscale at the 4th exposure. It is clear that the use of the present invention considerably increases the speed with which an X-ray image with accurate brightness can be obtained.

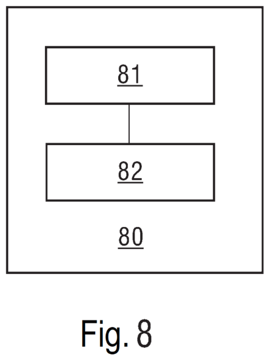

Fig. 8 is a structural schematic drawing of a tube voltage adjustment apparatus 80 provided in embodiments of the present invention. As shown in Figure 8, the apparatus 80 mainly comprises: an image grayscale acquisition module 81 and a tube voltage adjustment module 82, wherein:
The image grayscale acquisition module 81 is configured to: at the end of a present exposure, obtain an X-ray image acquired by the present exposure, obtain an average grayscale of the X-ray image, and obtain a first ratio between the average grayscale and a target grayscale of this X-ray examination.

The tube voltage adjustment module 82 is configured to: obtain an adjustment direction and an adjustment extent of tube voltage of an X-ray tube according to the first ratio; and adjust a present tube voltage according to the adjustment direction and adjustment extent of tube voltage of the X-ray tube.

In an optional embodiment, the image grayscale acquisition module 81 obtaining an average grayscale of the X-ray image comprises: removing pixel points lower than a first grayscale value and higher than a second grayscale value in the X-ray image, calculating an average grayscale of the remaining pixel points, and taking the average grayscale thus obtained to be the average grayscale of the X-ray image, wherein the first grayscale is the grayscale of a pixel point whose sequenced position is equal to an a% position when all pixel points of the X-ray image are sequenced from low to high grayscale, and the second grayscale is the grayscale of a pixel point whose sequenced position is equal to a b% position when all pixel points of the X-ray image are sequenced from low to high grayscale; a and b are preset values, and a < b.

In an optional embodiment, the tube voltage adjustment module 82 obtaining an adjustment direction and an adjustment extent of tube voltage of an X-ray tube according to the first ratio comprises: when the first ratio is greater than 1, determining the adjustment direction of tube voltage of the X-ray tube to be: decreasing the present tube voltage; when the first ratio is less than 1, determining the adjustment direction of tube voltage of the X-ray tube to be: increasing the present tube voltage; and the greater the distance between the first ratio and 1, the greater the adjustment extent of the present tube voltage.

In an optional embodiment, the tube voltage adjustment module 82 obtaining an adjustment direction and an adjustment extent of tube voltage of an X-ray tube according to the first ratio comprises: performing a logarithm operation on the first ratio, and obtaining an adjustment step length of the present tube voltage according to the principle that an adjustment step length of tube voltage and a result of the logarithm operation on the first ratio form an inverse linear relationship; or comprises: calculating a second ratio between a set tube current of the present exposure and an actual tube current of the present exposure, performing a logarithm operation on the product of the first ratio and the second ratio, and obtaining an adjustment step length of the present tube voltage according to the principle that an adjustment step length of tube voltage and a result of the logarithm operation on the product form an inverse linear relationship.

In an optional embodiment, the tube voltage adjustment module 82 obtaining an adjustment direction and an adjustment extent of tube voltage of an X-ray tube according to the first ratio comprises: obtaining a present base adjustment extent according to the present tube voltage, obtaining a present correction factor according to the first ratio, and obtaining an adjustment step length of the present tube voltage according to the present base adjustment extent and the present correction factor.

In an optional embodiment, the tube voltage adjustment module 82 obtaining a present base adjustment extent according to the present tube voltage comprises: if the present tube voltage is less than a first voltage threshold, calculating the difference between the present tube voltage and a tube voltage lower limit less 1, and obtaining the present base adjustment extent according to the principle that the present base adjustment extent and a result of a logarithm operation on the difference form a positive linear relationship; if the present tube voltage is greater than or equal to the first voltage threshold and less than a second voltage threshold, obtaining the present base adjustment extent according to the principle that the present base adjustment extent and the present tube voltage form a positive linear relationship; if the present tube voltage is greater than or equal to the second voltage threshold, taking the difference between a tube voltage upper limit and the second voltage threshold to be the present base adjustment extent.

In an optional embodiment, the tube voltage adjustment module 82 obtaining a present correction factor according to the first ratio comprises: performing a logarithm operation on the first ratio, and obtaining the present correction factor according to the principle that the present correction factor and a result of the logarithm operation on the first ratio form an inverse linear relationship; or comprises: calculating a second ratio between a set tube current of the present exposure and an actual tube current of the present exposure, performing a logarithm operation on the product of the first ratio and the second ratio, and obtaining the present correction factor according to the principle that the present correction factor and a result of the logarithm operation on the product form an inverse linear relationship.

In an optional embodiment, the tube voltage adjustment module 82 obtaining an adjustment step length of the present tube voltage according to the present base adjustment extent and the present correction factor comprises: taking the product of the present base adjustment extent and the present correction factor to be the adjustment step length of the present tube voltage.

In an optional embodiment, after the end of the present exposure, before obtaining the average grayscale of the X-ray image, the image grayscale acquisition module 81 is further configured to: judge whether the first ratios corresponding to a preset number of most recent consecutive exposures are all within [1 - *c%,* 1 + *c*%], and if so, determine that a tube voltage adjustment process for this X ray examination ends, wherein *c* is a preset value.

Embodiments of the present invention further provide an X-ray system, comprising the tube voltage adjustment apparatus 80 as described in any one of the embodiments above.

It should be explained that the tube voltage adjustment method and apparatus and the X-ray system provided in embodiments of the present invention may be a method, an apparatus and a system used in medical imaging.

The X-ray system in embodiments of the present invention may be a C-arm X-ray system, e.g. a mobile C-arm X-ray system.

Embodiments of the present invention further provide a computer program product, comprising a computer program or instruction, which, when executed by a processor, implements the steps of the tube voltage adjustment method as described in any one of the above embodiments.

Embodiments of the present invention further provide a computer-readable storage medium, storing an instruction which, when executed by a processor, can execute the steps of the tube voltage adjustment method as described above. In practical applications, the computer-readable medium may be included in each device/apparatus/system in the above embodiments, or may exist independently without being fitted into the device/apparatus/system. The computer-readable storage medium stores an instruction which, when executed by a processor, can execute the steps of the tube voltage adjustment method as described above.

Embodiments of the present invention further provide an electronic device. The electronic device may comprise a processor with one or more processing cores, a memory with one or more computer-readable storage media, and a computer program stored in the memory and executable on the processor. When the program in the memory is executed, the tube voltage adjustment method described above can be implemented.

Those skilled in the art will understand that features stated in the various embodiments and/or claims disclosed in the present application can be combined and/or integrated in various ways, even if such combinations or integrations are not clearly stated in the present application. In particular, without departing from the spirit and teaching of the present application, features stated in the various embodiments and/or claims of the present application can be combined and/or integrated in various ways, and all such combinations and/or integrations fall within the scope of disclosure of the present application.

Specific embodiments have been used herein to expound the principles and implementations of the present application, but the description of the embodiments above is merely intended to assist understanding of the method of the present application and the core idea thereof, not to restrict the present application. Those skilled in the art could make changes to specific implementations and scope of application, based on the idea, spirit and principles of the present application, and any changes, equivalent replacements, improvements, etc. made by those skilled in the art should be included within the scope of protection of the present application.

## Claims

1. A tube voltage adjustment method, **characterized in that** the method comprises:
at the end of a present exposure, obtaining an X-ray image acquired by the present exposure, and obtaining an average grayscale of the X-ray image;
obtaining a first ratio between the average grayscale and a target grayscale of this X-ray examination;
obtaining an adjustment direction and an adjustment extent of tube voltage of an X-ray tube according to the first ratio;
adjusting a present tube voltage according to the adjustment direction and adjustment extent of tube voltage of the X-ray tube.

2. The method as claimed in claim 1, **characterized in that** the step of obtaining an average grayscale of the X-ray image comprises:
removing pixel points lower than a first grayscale value and higher than a second grayscale value in the X-ray image, calculating an average grayscale of remaining pixel points, and taking the average grayscale thus obtained to be the average grayscale of the X-ray image;
wherein the first grayscale is the grayscale of a pixel point whose sequenced position is equal to an a% position when all pixel points of the X-ray image are sequenced from low to high grayscale, and the second grayscale is the grayscale of a pixel point whose sequenced position is equal to a b% position when all pixel points of the X-ray image are sequenced from low to high grayscale; a and b are preset values, and a < b.

3. The method as claimed in claim 1, **characterized in that** the step of obtaining an adjustment direction and an adjustment extent of tube voltage of an X-ray tube according to the first ratio comprises:
when the first ratio is greater than 1, determining the adjustment direction of tube voltage of the X-ray tube to be: decreasing the present tube voltage;
when the first ratio is less than 1, determining the adjustment direction of tube voltage of the X-ray tube to be: increasing the present tube voltage;
and the greater the distance between the first ratio and 1, the greater the adjustment extent of the present tube voltage.

4. The method as claimed in claim 1, **characterized in that** the step of obtaining an adjustment direction and an adjustment extent of tube voltage of an X-ray tube according to the first ratio comprises:
performing a logarithm operation on the first ratio, and obtaining an adjustment step length of the present tube voltage according to the principle that an adjustment step length of tube voltage and a result of the logarithm operation on the first ratio form an inverse linear relationship;
or comprises:
calculating a second ratio between a set tube current of the present exposure and an actual tube current of the present exposure, performing a logarithm operation on the product of the first ratio and the second ratio, and obtaining an adjustment step length of the present tube voltage according to the principle that an adjustment step length of tube voltage and a result of the logarithm operation on the product form an inverse linear relationship.

5. The method as claimed in claim 1, **characterized in that** the step of obtaining an adjustment direction and an adjustment extent of tube voltage of an X-ray tube according to the first ratio comprises:
obtaining a present base adjustment extent according to the present tube voltage, obtaining a present correction factor according to the first ratio, and obtaining an adjustment step length of the present tube voltage according to the present base adjustment extent and the present correction factor.

6. The method as claimed in claim 5, **characterized in that** the step of obtaining a present base adjustment extent according to the present tube voltage comprises:
if the present tube voltage is less than a first voltage threshold, calculating the difference between the present tube voltage and a tube voltage lower limit less 1, and obtaining the present base adjustment extent according to the principle that the present base adjustment extent and a result of a logarithm operation on the difference form a positive linear relationship;
if the present tube voltage is greater than or equal to the first voltage threshold and less than a second voltage threshold, obtaining the present base adjustment extent according to the principle that the present base adjustment extent and the present tube voltage form a positive linear relationship;
if the present tube voltage is greater than or equal to the second voltage threshold, taking the difference between a tube voltage upper limit and the second voltage threshold to be the present base adjustment extent.

7. The method as claimed in claim 5, **characterized in that** the step of obtaining a present correction factor according to the first ratio comprises:
performing a logarithm operation on the first ratio, and obtaining the present correction factor according to the principle that the present correction factor and a result of the logarithm operation on the first ratio form an inverse linear relationship;
or comprises:
calculating a second ratio between a set tube current of the present exposure and an actual tube current of the present exposure, performing a logarithm operation on the product of the first ratio and the second ratio, and obtaining the present correction factor according to the principle that the present correction factor and a result of the logarithm operation on the product form an inverse linear relationship.

8. The method as claimed in any one of claims 5 - 7, **characterized in that** the step of obtaining an adjustment step length of the present tube voltage according to the present base adjustment extent and the present correction factor comprises:
taking the product of the present base adjustment extent and the present correction factor to be the adjustment step length of the present tube voltage.

9. The method as claimed in claim 1, **characterized in that** after the end of the present exposure, before the step of obtaining the average grayscale of the X-ray image, the method further comprises:
judging whether the first ratios corresponding to a preset number of most recent consecutive exposures are all within [1 - *c%,* 1 + *c*%], and if so, determining that a tube voltage adjustment process for this X ray examination ends, wherein *c* is a preset value.

10. A tube voltage adjustment apparatus, **characterized in that** the apparatus comprises:
an image grayscale acquisition module, configured to: at the end of a present exposure, obtain an X-ray image acquired by the present exposure, obtain an average grayscale of the X-ray image, and obtain a first ratio between the average grayscale and a target grayscale of this X-ray examination;
a tube voltage adjustment module, configured to: obtain an adjustment direction and an adjustment extent of tube voltage of an X-ray tube according to the first ratio; and adjust a present tube voltage according to the adjustment direction and adjustment extent of tube voltage of the X-ray tube.

11. The apparatus as claimed in claim 10, **characterized in that** the tube voltage adjustment module obtaining an adjustment direction and an adjustment extent of tube voltage of an X-ray tube according to the first ratio comprises:
when the first ratio is greater than 1, determining the adjustment direction of tube voltage of the X-ray tube to be: decreasing the present tube voltage;
when the first ratio is less than 1, determining the adjustment direction of tube voltage of the X-ray tube to be: increasing the present tube voltage;
and the greater the distance between the first ratio and 1, the greater the adjustment extent of the present tube voltage.

12. The apparatus as claimed in claim 10, **characterized in that** the tube voltage adjustment module obtaining an adjustment direction and an adjustment extent of tube voltage of an X-ray tube according to the first ratio comprises:
performing a logarithm operation on the first ratio, and obtaining an adjustment step length of the present tube voltage according to the principle that an adjustment step length of tube voltage and a result of the logarithm operation on the first ratio form an inverse linear relationship;
or comprises:
calculating a second ratio between a set tube current of the present exposure and an actual tube current of the present exposure, performing a logarithm operation on the product of the first ratio and the second ratio, and obtaining an adjustment step length of the present tube voltage according to the principle that an adjustment step length of tube voltage and a result of the logarithm operation on the product form an inverse linear relationship.

13. The apparatus as claimed in claim 10, **characterized in that** the tube voltage adjustment module obtaining an adjustment direction and an adjustment extent of tube voltage of an X-ray tube according to the first ratio comprises:
obtaining a present base adjustment extent according to the present tube voltage, obtaining a present correction factor according to the first ratio, and obtaining an adjustment step length of the present tube voltage according to the present base adjustment extent and the present correction factor.

14. The apparatus as claimed in claim 13, **characterized in that** the tube voltage adjustment module obtaining a present base adjustment extent according to the present tube voltage comprises:
if the present tube voltage is less than a first voltage threshold, calculating the difference between the present tube voltage and a tube voltage lower limit less 1, and obtaining the present base adjustment extent according to the principle that the present base adjustment extent and a result of a logarithm operation on the difference form a positive linear relationship;
if the present tube voltage is greater than or equal to the first voltage threshold and less than a second voltage threshold, obtaining the present base adjustment extent according to the principle that the present base adjustment extent and the present tube voltage form a positive linear relationship;
if the present tube voltage is greater than or equal to the second voltage threshold, taking the difference between a tube voltage upper limit and the second voltage threshold to be the present base adjustment extent.

15. The apparatus as claimed in claim 13 or 14, **characterized in that** the tube voltage adjustment module obtaining a present correction factor according to the first ratio comprises:
performing a logarithm operation on the first ratio, and obtaining the present correction factor according to the principle that the present correction factor and a result of the logarithm operation on the first ratio form an inverse linear relationship;
or comprises:
calculating a second ratio between a set tube current of the present exposure and an actual tube current of the present exposure, performing a logarithm operation on the product of the first ratio and the second ratio, and obtaining the present correction factor according to the principle that the present correction factor and a result of the logarithm operation on the product form an inverse linear relationship.

16. The apparatus as claimed in claim 10, **characterized in that** after the end of the present exposure, before obtaining the average grayscale of the X-ray image, the image grayscale acquisition module is further configured to:
judge whether the first ratios corresponding to a preset number of most recent consecutive exposures are all within [1 - c%, 1 + c%], and if so, determine that a tube voltage adjustment process for this X ray examination ends, wherein c is a preset value.

17. An X-ray system, **characterized in that** the system comprises the tube voltage adjustment apparatus as claimed in any one of claims 10 - 16.

18. A computer-readable storage medium, **characterized in that** the computer-readable storage medium stores an instruction which, when executed by a processor, executes the steps of the tube voltage adjustment method as claimed in any one of claims 1 - 9.
